# EUROPEAN PATENT SPECIFICATION

(11) **EP 2 716 212 B1**
(45) Date of publication and mention of the grant of the patent: **31.08.2016**
(21) Application number: 12761386.7
(22) Date of filing: 23.03.2012
(51) Int. Cl.: A61B 5/11, G01B 7/24, G01L 1/12, A61C 19/04, G01L 1/04, A61B 5/22

(54) **DEVICE FOR TRACKING MASTICATORY MOVEMENTS**
VORRICHTUNG ZUR VERFOLGUNG VON KAUBEWEGUNGEN
DISPOSITIF POUR SURVEILLER LA MASTICATION

(30) Priority: 24.03.2011 RU 2011111125
(43) Date of publication of application: 09.04.2014
(73) Proprietor: Magomedov, Magomed Habibovich, Moscow 119330 (RU)
(72) Inventor: Magomedov, Magomed Habibovich, Moscow 119330 (RU)
(74) Representative: Klemm, Rolf
(86) International application number: PCT/RU2012/000207
(87) International publication number: WO 2012/128669

(56) References cited:
- FR-A1- 2 741 258
- RU-C1- 2 348 355
- RU-C2- 2 244 309
- US-A- 4 765 345
- US-A1- 2006 064 037
- US-A1- 2008 146 890

## Description

### Field of technology

The invention relates to measuring devices in biomedical technology for studies of movement of a human body or its parts for diagnostic purposes, and more particularly to devices for tracking of human masticatory movements.

### Background

Devices to track facial muscles of a human are known, see patent documents: EP1516586, JP2006129885, US6893406, and US7421233.

Devices to track masticatory movements are known as well, see patent documents: JP10011560, JP7171136, JP11123185, JP2001178706, JP2004242893, JP2004242902, and JP2005323213. These known devices are the closest analogs of the first and second embodiments of the claimed device. The drawback of these devices is that they are uncomfortable to use in day-to-day life.

FR 2 741 258 A1 describes a diagnostic and therapeutic equipment consisting of two sensors, which are used externally and situated, so that they may detect movements and particularly contractions of the jaw muscles and the oral floor muscles respectively. Additional sensors are provided to detect contractions of the lips and breath. A frame secured by head straps is used to support the first two sensors and the electrical signals produced from all the sensors are sent to a means of processing. These means include a screen which shows the movement recorded by the sensors against a pictorial background.

Known are the earphones with round housing, which embrace an auricle of an ear when a user puts it on and adjoins to the auricle and to the head, for instance see WO 2009/004401 of 08.01.2009 that is an analog of the claimed earphone. However, the main disadvantage is the lack of possibility to monitor the user's masticatory movements.

US 2006/0064037 A1 discloses au earphone according to the preamble of claim 1.

### Disclosure of the Invention

The invention is defined in the independent claim.

The technical result achieved in the claimed device for tracking masticatory movements, as well as in the claimed earphone, consists in an improvement of filtering quality, i.e. with the allocation of chewing movements from other user movements, such as head movements or jaw movements during a conversation, improvement of quality of conversion chewing and/or temporal muscles into electrical signals, as well as of their registration.

This, in particular, is achieved by the use of the housing in the claimed device the housings of the other known devices, such as earphones (in particular those that embrace an auricle of an ear or those that are implemented into the auricle of an ear or get attached to the auricle of an ear or the head of a user in the area of its temporal and/or chewing masticatory), comprising a housing adapted to be elastically deformed when there is a need.

One more technical result achieved by the claimed device to track masticatory movements and the earphone, is the correction of the threshold value for every cycle of mastication-chewing for its best use, taking into account the composition of the food eaten. This result is important because the frequency and the amplitude of the signal i.e. the chewing quality depends upon chewing of the food consistency: whether it is soft or solid food, meat or soup, etc.

The specified technical result is achieved in the device for tracking masticatory movements with at least one Hall sensor and at least one permanent magnet installed in the housing, with ability of their mutual displacement and information generation, as well as a control and information processing unit, which allows, in particular, counting chewing movements in number.

The elastically deformable housing may be implemented to be installed on the user's head and adjoining to the user's temporal and/or chewing muscles.

The elastically deformable housing may be implemented to be installed or fit in the user's auricle of an ear or embrace it.

The elastically deformable housing may be the housing of an earphone.

The elastically deformable housing may comprise of at least two parts connected elastically to each other, whereby on one of the housing parts the Hall sensor is installed and on the other part permanent magnet is installed.

The elastically deformable housing may comprise of at least two inelastic parts connected to each other, whereby on one of the housing parts the Hall sensor is installed and on the other part permanent magnet is installed.

The housing may be provided with a wall may be implemented as elastically deformable and a permanent magnet may be mounted on this wall.

The device may include at least one speaker key to set an initial threshold value of the number of chewing movements in each cycle of mastication and a LCD (liquid crystal display) screen to display a specified threshold value, and the control and information processing unit is adapted to detect deviations from the number of chewing movements predetermined by the threshold value and transmitting an audio message to the user through at least one speaker when the threshold value is reached.

The control and information processing unit may be configured to adjust a pre-set threshold value of chewing movements depending on the amount of chewing time per unit of mystification and of the intensity (amplitude) of the chewing movements. It should be clarified that the monitoring devices required for measuring the chewing movements (threshold value) can be set individually for each chewing cycle. The control and information processing unit determines the frequency and intensity (amplitude) of chewing movements depending on the consistency of the food. For solid foods (meat, bread etc.) and soft foods (soup broth, salad, etc.) the frequency and amplitude of chewing is different. Thus, analyses of these differences can change the defined (required) chewing threshold value up or down, adapted for each cycle of chewing food.

The device may be implemented with an interface configured for wired (e.g. USB) or wireless (e.g. Bluetooth) communication to transmit information for external devices or to exchange information with external devices.

The specified technical result is achieved by a device for tracking masticatory movements that allows counting the number of masticatory movements and is implemented with an elastic arch embraced to or pressed against the user's head or neck to be installed and fixed on the user's head in the area of temporal and/or chewing musculature. This result is achieved due to at least one Hall sensor and at least one permanent magnet installed with ability of mutual displacement in one elastically deformable body member as a first housing that is pressed by the arch to the user's head in the area of temporal and/or chewing musculature. The device also includes a control and information processing unit for data generated by mutual displacement of the Hall sensor and the permanent magnet due to the first housing or its parts deformation.

The elastically deformable first housing may comprise of at least two parts connected elastically to each other, one with the Hall sensor installed and the other with permanent magnet.

The elastically deformable first housing may comprise of at least two inelastic parts connected to each other, one with the Hall sensor installed and the other with permanent magnet.

The part of the housing wall may be implemented as elastically deformable and a permanent magnet wall may be installed on it.

In addition, the device may include a second elastically deformable housing, which is pressed by the arch to the user head, with installed in it at least one Hall sensor and at least one permanent magnet that may displace relative to each other when there is an elastic deformation of the housing. The control and information processing unit is implemented with ability to process signals from the Hall sensors.

The specified technical result is achieved in an earphone with a speaker that is applied to an ear or put on it because its housing is made, at least particularly, with ability of elastic deformation and includes at least one Hall sensor and one permanent magnet installed inside with ability of mutual displacement due to deformation of the housing or its parts, as well as a control and information processing unit connected with the speaker to process data induced by this mutual displacement allowing the user to monitor the process of chewing food.

The housing may comprise of at least two parts connected elastically to each other, one with the Hall sensor installed and the other with permanent magnet.

The control and information processing unit may be installed in the housing.

The earphone may additionally include at least one key to set an initial/pre-set threshold value for a number of chewing movements in each cycle of mastification and a LCD screen to show the configured threshold value that are installed on the earphone housing and connected with the control and information processing unit.

The control and information processing unit can be configured to adjust a pre-set threshold value of chewing movements depending on the amount of chewing time per unit and the amplitude (intensity) of chewing movements.

### Brief description of the drawings

Figure 1 presents a general view of the device for tracking masticatory movements that is implemented as an earphone;
Figure 2 shows a projection of the device for tracking masticatory movements;
Figure 3 shows a cross section of the device for tracking masticatory movements according to

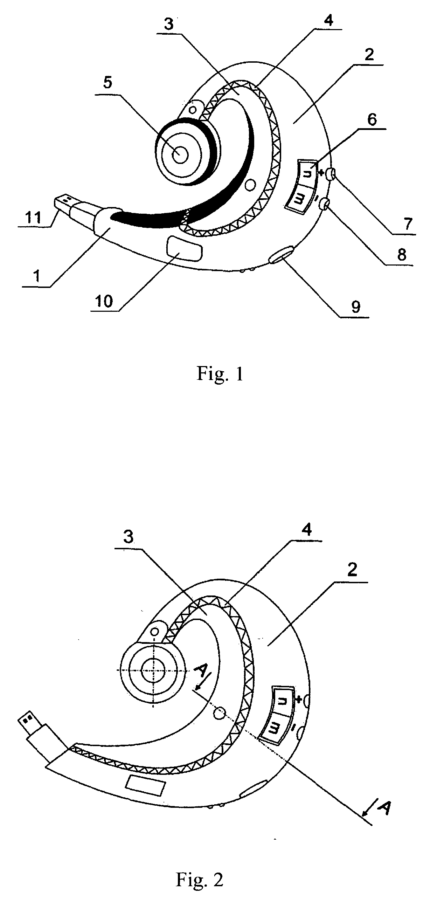

Figure 2, presenting the positions of the control board, the Hall sensors, and the permanent magnets in the housing;

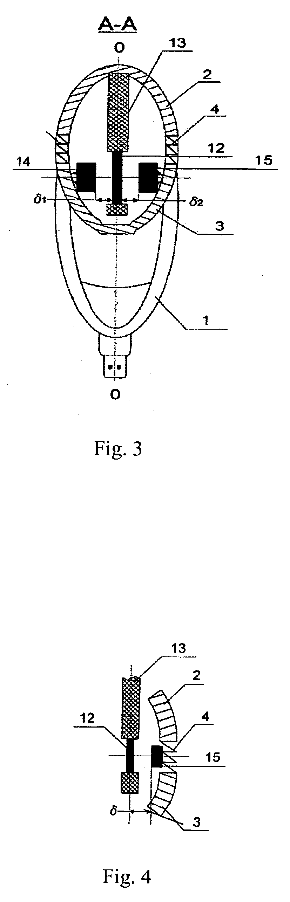

Figure 4 presents a configuration of the Hall sensor and the permanent magnet in the housing when the magnet is fixed on an elastic ring.

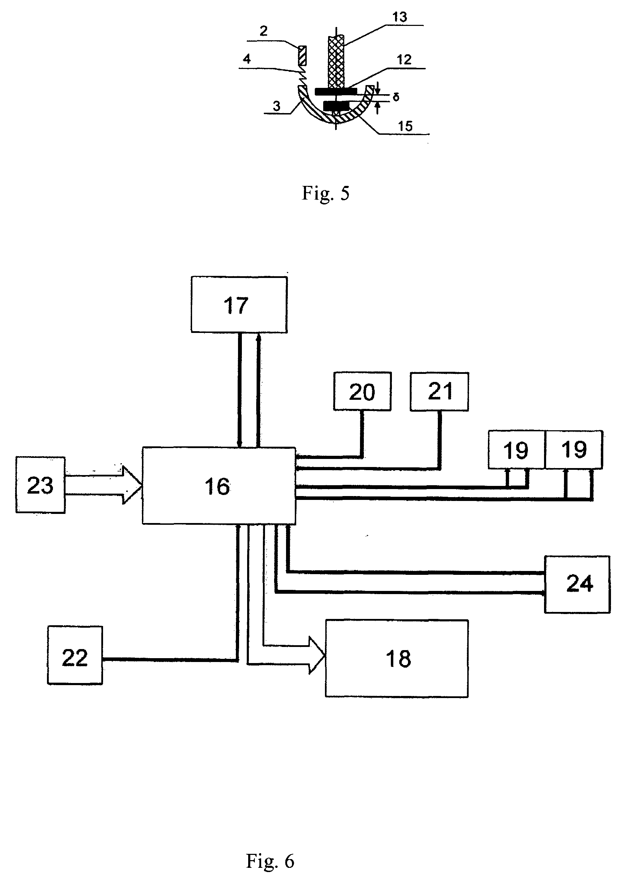

Figure 5 shows a cross section of the sample device for tracking masticatory movements, which shows the Hall sensor and magnet disposition perpendicularly the control board;
Figure 6 presents a flow chart of the device for tracking masticatory movements.

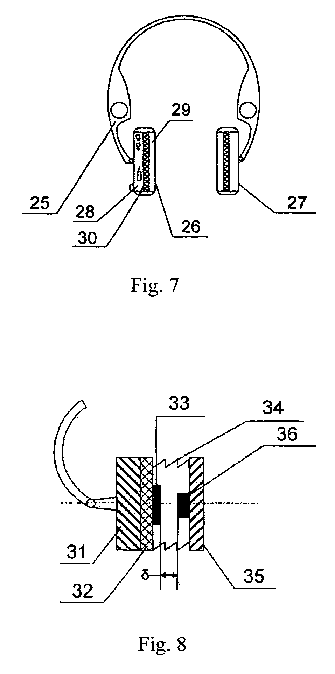

Figure 7 shows the device for tracking masticatory movements that is implemented with the elastic arch and includes two housings (in particular, for earphones), pressed by the arch to the user head;
Figure 8 presents a cross section of one housing of the device for tracking masticatory movements implemented with the elastic arch;

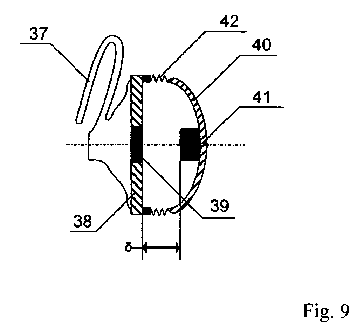

Figure 9 shows the device for tracking masticatory movements that is implemented to be inserted in the auricle and includes the elastic housing and the clamping element.

### Embodiments of the invention

As shown in Fig. 1, a device for tracking masticatory movements is implemented as an earphone with a housing 1 consisting of a upper part 2 and a lower part 3 that are separated by an elastic part, for example, an elastic ring 4. The housing and its components may be made of plastic or plexiglas, and of acryl, polystyrene, polyethylene, ABC, etc. The plastic may be rubberized. The housing or its parts may be of any color, they may be even transparent. The elastic part with spring properties may be made of any material, metallic or non-metallic, with elastic properties. In addition, ability to use these materials in combination with each other is not excluded. By its nature the elastic part should be of the specified rigidity and properties of multiple-spring action and its design may include springs of cylindrical, flat or conical shape. The spring may be made of steel, brass, bronze, etc., but rubber, reinforced plastic or a special metal alloy may be used too.

By its shape the elastic coupling can also be made as a narrow flat strip, a spiral, a rod, a membrane, a bellow or a tubular spring that can compress, stretch, bend, twist or perform combination of these movements simultaneously.

The membrane is thin elastic, mostly round, flat or corrugated plate with fixed edges. It may be metallic or non-metallic. Rubber, rubberized silk and leather may be used as material for non-metallic membranes. When the non-metallic membrane is used to connect the device housing parts it is recommended to use additionally a cylindrical or helical spring that creates the main counter-force when chewing.

The earphone is equipped with the speaker 5, the LCD 6, and keys 7 and 8 to install the threshold value for the chewing movements number in the chewing cycle indicated in the LCD screen 6. Figure 1 also shows positions of the switch 9, the battery pack 10, and the USB port 11. Figure 2 presents projection of the earphone, which general view is shown on the Fig. 1.

Inside the earphone the Hall sensor 12 is installed on the control board 13 attached to the housing 2, as it is shown on Fig. 3. The magnets 14 and 15 are fixed to the housing bottom walls 3 opposite to the Hall sensor 12 with gaps δ₁ and δ₂, respectively, with respect to it. It should be noticed that if the Hall sensor 12 is situated on the control board 13 the magnets 14 and 15 may be installed on the elastic housing part, for example, on the elastic ring 4, as it is shown on Fig. 4.

Figure 5 shows an example embodiment of disposition in the elastic housing of the magnet 15 and the Hall sensor 12, which is attached to the control board 13 perpendicularly to its axis O-O.

To track chewing movements the device is equipped with the control and information processing unit in the form of the microcontroller 16, which, as it is shown in Fig. 6, is connected with the Hall sensor 17 (12 in Fig. 3), the speaker 18 (5 in Fig. 1), the LCD screen 19 (6 in Fig. 1), and keys 20 and 21 (7 and 8 in Fig. 1), as well as with the switch 22 (9 on Fig. 1), the battery pack 23 (10 in Fig. 1), and the USB port 24 (11 in Fig. 1).

When the user puts on the earphone shown on Fig. 1, its housing bottom part 2 adjoins to the user's auricle and/or head in the area of the temporal and/or chewing musculature. When chewing, the bottom part 3 of the housing moves due to temporal and chewing muscles action and oscillates relatively the top part 2 of the housing due to its inertia and due to the elastic ring 4 connecting parts 2 and 3 of the housing. As a result of the housing parts 2 and 3 interaction the magnets 14 and 15 are displaced relative to Hall sensor 12. This induces a signal proportional to the distance change of the gaps δ₁ and δ₂. The microcontroller 16 (Fig. 6) that is the part of the control and information processing unit counts the number of these signals corresponding to the movements in every chewing cycle, compares the obtained value with the current corrected threshold value given via keys 20 and 21 (7 and 8 in Fig. 1) and, if the current chewing movements number equals to the threshold value, issues to the user with the speaker 18 (5 in Fig. 1 and Fig. 2) a sound command to ingest food.

Figure 7 shows the second option of the tracking device for chewing movements that is implemented with the elastic arch 25 and includes two housings 26 and 27, pressed to the user's head by the arch 25. In particular, the second embodiment of the device for tracking masticatory movements may be implemented in the form of earphones connected with the elastic arch. Each of the housings 26 and 27 consists of two parts 28 and 29 connected by the elastic ring 30. In detail the housing 26 construction is shown in Fig. 8 where one part of the housing consists of the platform 31 and the plate 32, on which the Hall sensor 33 is installed. The second part of the housing (the plate 35 with the magnet 36 installed on it) is connected with the plate 32 by the elastic ring 34. The device may be equipped with a speaker, a LCD screen, and keys to set a threshold value of masticatory movements in one chewing cycle, which is displayed on the LCD screen installed in one of the housings along with the processor, the switch, the battery pack and the USB port. Due to interaction of the housing parts the magnets oscillate relatively the Hall sensor inducing signal processed by the microcontroller that counts the oscillation number (the number of chewing movements in every chewing cycle), compares the obtained value with the current corrected threshold value, and, if the current chewing movements number equals to the threshold value given via keys 20 and 21 (7 and 8 in Fig. 1 and Fig. 2), issues to the user with the speaker 18 (5 in Fig. 1 and Fig. 2) a sound command to ingest food. Then a new food chewing cycle begins.

Figure 9 shows the device for tracking masticatory movements with the elastically deformable housing, which design allows its installation in the user's auricle. The device includes the elastic arch 37, which is designed to embrace the auricle and connected with the housing consisting of two parts. The first part is the plate 38 with the Hall sensor 39 on it. The second part 40 with the magnet 41 installed on it is connected with the first part via an elastic ring 42.. The device may be equipped with a speaker, a LCD screen, and keys to set a threshold value of masticatory movements in one chewing cycle, which is indicated on the LCD screen installed in one of the housings along with the processor, a switch, a battery pack and a USB port. Due to interaction of the housing parts the magnets oscillate relative to the Hall sensor inducing signal processed by the microcontroller that counts the oscillation number (the number of chewing movements in every chewing cycle), compares the obtained value with the value specified by keys 20 and 21 (7 and 8 in Fig. 1 and Fig. 2) or with the corrected threshold value, and, if the current chewing movements number equals to the threshold value, issues to the user a sound command with the speaker 18 (5 in Fig. 1 and Fig. 2) to ingest food. Then a new food chewing cycle begins.

The implementation of the device for tracking masticatory movements and the earphone with the LCD screen to indicate the threshold value for chewing movements number was described above. The option to implement these devices without the LCD indicator is not excluded. In this case the information about the threshold value for chewing movements number stored in the microcontroller memory is reported to the user by the voice or sound signal during the device turning-on. Then the user increases or reduces the threshold value for the number of chewing movements with keys 7 or 8, and the current threshold value is announced to the user with each key press. This approach decreases the cost of the device for tracking masticatory movements and the earphone.

## Claims

1. An earphone to track masticatory movements comprising a housing (1, 26) made to be elastically deformable, a speaker (5, 18), and a control and information processing unit (13, 16) connected with the speaker **characterised by** a Hall sensor (12, 33, 39) and a permanent magnet (14, 15, 36, 41) whereby the Hall sensor and the permanent magnet are installed inside the housing with ability of mutual displacement during the housing deformation.

2. The earphone according claim 1, **characterized in that** its housing is made as elastically deformable with ability to embrace a user's auricle and to contact with his/her temporal and/or chewing muscles.

3. The earphone according claim 1, **characterized in that** its housing is made as elastically deformable with ability of installation in a user's auricle.

4. The earphone according claim 1, **characterized in that** the housing comprises at least two parts (2, 3, 28, 29, 38, 40) elastically connected with each other, whereby the Hall sensor is installed on one (2, 28, 38) of the at least two parts and the permanent magnet is installed on an other one (3, 29, 40) of the at least two parts.

5. The earphone according to one of the claims 1 to 4, **characterized in that** the housing is formed with an elastically deformable wall, on which the permanent magnet is installed.

6. The earphone according to one of the claims 1 to 5, **characterized in that** the control and information processing unit is installed in the elastically deformable housing,

7. The earphone according to one of the claims 1 to 6, **characterized in that**, the earphone further includes at least one key (7, 8, 20, 21) to set a threshold value for a number of chewing movements in each chewing cycle, and a LCD screen (6, 19) to display the set threshold value, wherein the at least one key and the LCD screen are installed on the elastically deformable housing and connected with the control and information processing unit.

8. The earphone according to one of the claims 1 to 7, **characterized in that** the control and information processing unit is configured to determine the frequency and amplitude of chewing movements and to adjust a pre-set threshold value for a number of chewing movements in each chewing cycle depending on the frequency and amplitude of the chewing movements.

9. The earphone according to one of claims 1 to 8, **characterized in that** it is implemented with an interface configured for wired or wireless communication with external devices.

10. A device for tracking masticatory movements comprising the earphone according to claim 1 and an elastic arch (25) configured to embrace a user's head or neck, to be installed and fixed on the user's head in the area of temporal and/or chewing musculature, and to press the earphone housing (26) to the user's head in the area of temporal and/or chewing musculature.

11. The device according claim 10, **characterized in that** it further comprises a second housing (27) elastically deformable, which is pressed by the arch (25) to the user's head, with installed in it at least one further Hall sensor (33) and at least one further permanent magnet (36) that may be displaced one relative to another, and the control and information processing unit is implemented with ability to process signals from the Hall sensors (33) from both housings (26, 27).

## Patentansprüche

1. Ohrhörer, um die mastikatorischen Bewegungen zu verfolgen, umfassend ein Gehäuse (1, 26), das hergestellt ist, um elastisch verformbar zu sein, einen Lautsprecher (5, 18) und eine Steuerung und eine Informationsverarbeitungseinheit (13, 16), die mit dem Lautsprecher (5, 18) verbunden ist, **gekennzeichnet durch** einen Hall-Sensor (12, 33, 39) und einen Permanentmagneten (14, 15, 36, 41), wobei der Hall-Sensor und der Permanentmagnet innerhalb des Gehäuses mit der Fähigkeit einer wechselseitigen Versetzung während der Gehäuseverformung installiert sind.

2. Ohrhörer nach Anspruch 1, **dadurch gekennzeichnet, dass** sein Gehäuse als elastisch verformbarer Körper mit der Fähigkeit hergestellt ist, die Aurikel des Anwenders zu umgreifen und mit seiner/ihrer Schläfe und/oder Kaumuskeln zu kontaktieren.

3. Ohrhörer nach Anspruch 1, **dadurch gekennzeichnet, dass** sein Gehäuse als elastisch deformierbarer Körper mit der Fähigkeit einer Installation in einem Aurikel von dem Anwender hergestellt ist.

4. Ohrhörer nach Anspruch 1, **dadurch gekennzeichnet, dass** das Gehäuse zumindest zwei Teile (2, 3, 28, 29, 38, 40) umfasst, die miteinander elastisch verbunden sind, wobei der Hall-Sensor in einem Teil (2, 28, 38) der zumindest zwei Teile installiert ist und der Permanentmagnet auf einem anderen von einem Teil (3, 29, 40) von den zumindest zwei Teilen installiert ist.

5. Ohrhörer nach einem der Ansprüche 1 bis 4, **dadurch gekennzeichnet, dass** das Gehäuse mit einem elastisch verformbaren Wand ausgebildet ist, auf welcher der Permanentmagnet installiert ist.

6. Ohrhörer nach einem der Ansprüche 1 bis 5, **dadurch gekennzeichnet, dass** die Steuerung und die Informationsverarbeitungseinheit in dem elastisch verformbaren Gehäuse installiert sind.

7. Ohrhörer nach einem der Ansprüche 1 bis 6, **dadurch gekennzeichnet, dass** der Ohrhörer zumindest eine Taste (7, 8, 20, 21) umfasst, um einen Grenzwert für eine Anzahl von Kaubewegungen in einem Kauzyklus festzusetzen und eine LCD-Bildanzeige (6, 19) um den gesetzten Grenzwert anzuzeigen, wobei die zumindest eine Taste und die LCD-Bildanzeige auf dem elastisch verformbaren Gehäuse installiert sind und mit der Steuerung und Informationsverarbeitungseinheit verbunden sind.

8. Ohrhörer nach einem der Ansprüche 1 bis 7, **dadurch gekennzeichnet, dass** die Steuerung und die Informationsverarbeitungsverarbeitungseinheit eingerichtet sind, um die Frequenz und die Amplitude der Kaubewegungen und einen vorhergesetzten Grenzwert für eine Anzahl von Kaubewegungen zu bestimmen.

9. Ohrhörer nach einem der Ansprüche 1 bis 8, **dadurch gekennzeichnet, dass** er mit einer Schnittstelle implementiert ist, die für eine kabelgebundene oder kabellose Kommunikation mit externen Vorrichtungen ausgebildet ist.

10. Vorrichtung zum Verfolgen von mastikatorischen Bewegungen umfassend den Ohrhörer nach Anspruch 1 und einen elastischen Bogen (25) der ausgebildet ist, um einen Kopf oder Hals eines Anwenders zu umklammern, um auf dem Kopf des Anwenders im Bereich von Schläfe und/oder der Kaumuskulatur installiert und befestigt zu werden und das Ohrgehäuse (26) an den Kopf des Anwenders im Bereich der Schläfe und/oder der Kaumuskulatur zu pressen.

11. Vorrichtung nach Anspruch 10, **dadurch gekennzeichnet, dass** sie ferner ein zweites elastisch deformierbares Gehäuse (27) umfasst, das mittels des Bogens (25) an den Kopf des Anwenders gepresst wird und das zumindest einen weiteren Hall-Sensor (33) und zumindest einen weiteren Permanentmagneten (36), darin installiert, aufweist, die relativ zueinander angeordnet werden können und die Steuerung und Informationsverarbeitungseinheit mit der Fähigkeit implementiert ist, Signale von den Hall-Sensoren (33) aus beiden Gehäusen (26, 27) zu verarbeiten.

## Revendications

1. Ecouteur pour suivre les mouvements de mastication comprenant un boîtier (1, 26) fabriqué de manière à être déformable élastiquement, un haut-parleur (5, 18) et une unité de commande et de traitement d'information (13, 16) connectée au haut-parleur,
**caractérisé par** :
un capteur Hall (12, 33, 39) et un aimant permanent (14, 15, 36, 41) de sorte que le capteur Hall et l'aimant permanent soient installés à l'intérieur du boîtier tout en disposant d'une capacité de déplacement mutuel pendant la déformation du boîtier.

2. Ecouteur selon la revendication 1, **caractérisé en ce que** son boîtier est fabriqué de manière à être déformable élastiquement tout en disposant de la capacité d'épouser la forme de l'oreille d'un utilisateur et d'entrer en contact avec ses muscles temporaux et/ou masticateurs.

3. Ecouteur selon la revendication 1, **caractérisé en ce que** son boîtier est fabriqué de manière à être déformable élastiquement tout en disposant de la capacité d'être installé dans l'oreille d'un utilisateur.

4. Ecouteur selon la revendication 1, **caractérisé en ce que** le boîtier comprend au moins deux parties (2, 3, 28, 29, 38, 40) connectées élastiquement l'une à l'autre ou les unes aux autres de sorte que le capteur Hall soit installé sur l'une (2, 28, 38) des au moins deux parties et que l'aimant permanent soit installé sur l'autre/une autre (3, 29, 40) des au moins deux parties.

5. Ecouteur selon l'une des revendications 1 à 4, **caractérisé en ce que** le boîtier est formé de manière à comporter une paroi déformable élastiquement sur laquelle l'aimant permanent est installé.

6. Ecouteur selon l'une des revendications 1 à 5, **caractérisé en ce que** l'unité de commande et de traitement d'information est installée dans le boîtier déformable élastiquement.

7. Ecouteur selon l'une des revendications 1 à 6, **caractérisé en ce que** l'écouteur inclut en outre au moins une touche (7, 8, 20, 21) afin d'établir une valeur de seuil pour un certain nombre de mouvements de mastication dans chaque cycle de mastication et un écran LCD (6, 19) afin d'afficher la valeur de seuil établie, dans lequel l'au moins une touche et l'écran LCD sont installés sur le boîtier déformable élastiquement et sont connectés avec l'unité de commande et de traitement d'information.

8. Ecouteur selon l'une des revendications 1 à 7, **caractérisé en ce que** l'unité de commande et de traitement d'information est configurée de manière à déterminer la fréquence et l'amplitude de mouvements de mastication et de manière à régler une valeur de seuil préétablie pour un certain nombre de mouvements de mastication dans chaque cycle de mastication en fonction de la fréquence et de l'amplitude des mouvements de mastication.

9. Ecouteur selon l'une des revendications 1 à 8, **caractérisé en ce qu'**il est mis en oeuvre avec une interface configurée pour une communication câblée ou sans fil avec des dispositifs externes.

10. Dispositif pour suivre des mouvements de mastication comprenant un écouteur selon la revendication 1 et un arceau élastique (25) configuré de manière à épouser la forme de la tête ou du cou d'un utilisateur, de manière à être installé et fixé sur la tête de l'utilisateur dans la zone de la musculature temporale et/ou de mastication et de manière à presser le boîtier d'écouteur (26) sur la tête de l'utilisateur dans la zone de la musculature temporale et/ou de mastication.

11. Dispositif selon la revendication 10, **caractérisé en ce qu'**il comprend en outre un second boîtier (27) déformable élastiquement, lequel est pressé par l'arceau (25) sur la tête de l'utilisateur, au moins un autre capteur Hall (33) et au moins un autre aimant permanent (36) qui peuvent être déplacés l'un par rapport à l'autre ou les uns par rapport aux autres étant installés dans ce boîtier, et l'unité de commande et de traitement d'information est mise en oeuvre tout en disposant de la capacité de traiter des signaux en provenance des capteurs Hall (33) installés dans les deux boîtiers (26, 27).
